# EUROPÄISCHE PATENTSCHRIFT

(11) **EP 2 254 664 B1**
(45) Veröffentlichungstag und Bekanntmachung des Hinweises auf die Patenterteilung: **24.04.2013**
(21) Anmeldenummer: 09710903.7
(22) Anmeldetag: 12.02.2009
(51) Int. Cl.: A61N 1/44, A61N 1/32, A61K 41/00, A61L 2/14, C12M 1/42, C12N 13/00, H05H 1/24

(54) **PLASMA-GERÄT ZUR SELEKTIVEN BEHANDLUNG ELEKTROPORIERTER ZELLEN**
PLASMA DEVICE FOR SELECTIVE TREATMENT OF ELECTROPORED CELLS
APPAREIL À PLASMA POUR LE TRAITEMENT SÉLECTIF DE CELLULES ÉLECTROPORÉES

(30) Priorität: 12.02.2008 DE 102008008614
(43) Veröffentlichungstag der Anmeldung: 01.12.2010
(73) Patentinhaber: INP Greifswald Leibniz-Institut für Plasmaforschung und Technologie e. V., 17489 Greifswald (DE)
(72) Erfinder: STIEBER, Manfred, 17489 Greifswald (DE); WELTMANN, Klaus-Dieter, 18609 Ostseebad Binz (DE); VON WOEDTKE, Thomas, 18519 Horst (DE); WILKE, Christian, 17489 Greifswald (DE); EHRENBERG, Uwe, 17509 Hanshagen (DE)
(74) Vertreter: Wehlan, Helmut
(86) Internationale Anmeldenummer: PCT/EP2009/051650
(87) Internationale Veröffentlichungsnummer: WO 2009/101143

(56) Entgegenhaltungen:
- DE-A1-102006 019 664
- DE-A1-102007 025 452
- DE-T2- 60 106 901
- US-A1- 2002 122 896
- US-A1- 2006 148 737
- US-A1- 2007 029 500
- US-A1- 2009 004 717

## Beschreibung

Die Erfindung betrifft ein Verfahren und Vorrichtungen zur Behandlung lebender Zellen mittels eines kalten Atmosphärendruckplasmas bei gleichzeitiger selektiver Elektroporation der Zellen zur lokalen, selektiven Abtötung von Krebszellen, der Verbesserung der Wundbehandlung und einer verbesserten antimikrobiellen Plasmawirkung.

### [Stand der Technik]

### 1. Problem der Krebstherapie:

Die einzeln oder in Kombination verwendeten Standardverfahren der Krebstherapie sind heute die Operation, die Chemotherapie und die Strahlungstherapie. In der klinischen Erprobung befindet sich derzeit eine Methode, die als "Elektroporationstherapie" (EPT), "Elektrochemotherapie" (ECT) bzw. "Hochspannungs-Impulstherapie" (HVIT) bezeichnet wird und darauf basiert, dass durch die gezielte Anwendung gepulster elektrischer Felder selektiv die Membranporen der Krebszellen kurzzeitig reversibel geöffnet werden (-Elektroporation), so dass eine drastisch erhöhte Aufnahme der für die Chemotherapie verwendeten Wirkstoffe, wie beispielsweise Bleomycin bzw. Cisplatin durch die Zellen erzielt wird. Die Vorteile gegenüber der konventionellen Chemotherapie bestehen darin, dass bei der Elektrochemotherapie die erforderliche Wirkstoffdosis etwa zwanzigfach niedriger gewählt werden kann, wegen der selektiven Abtötung eine Gewebe schonende Wirkung bei minimaler Narbenbildung und eine starke Reduzierung der durch das Chemotherapeutikum hervorgerufenen Nebenwirkungen erreicht wird. Da sich die Krebszellen von den gesunden Zellen hinsichtlich ihrer Größe, Struktur der Zellmembran und der elektrischen Eigenschaften unterscheiden, kann eine Selektivität der Elektroporation durch eine geeignete Wahl der Amplitude, Anzahl, Dauer und Frequenz der Hochspannungsimpulse erreicht werden. In den Patentschriften DE69604509, DE69928383 und DE60106901 werden verschiedene Vorrichtungen dafür beschrieben, eine solche gesteuerte, selektive Elektroporation von Zellen zu realisieren.

Auch wenn bei der Elektrochemotherapie die Wirkstoffdosis beträchtlich reduziert werden kann, so kann doch nicht völlig auf den Einsatz teurer Chemotherapeutika verzichtet werden, die, zwar in geringerem Maße als bei konventioneller Chemotherapie, Nebenwirkungen auf gesundes Körpergewebe haben.

### 2. Problem der Heilung chronischer Wunden:

Da chronische Wunden meist die Folge von Grunderkrankungen sind, besteht der primäre Ansatz für ihre Heilung in der Diagnostik und Kausaltherapie dieses Grundleidens. In vielen Fällen ist die Heilung der Grunderkrankung jedoch nicht möglich, so dass durch vielfältige lokaltherapeutische Maßnahmen, wie beispielsweise durch chirurgische Eingriffe, Wundreinigung und -desinfektion, durch den Einsatz von Antibiotika sowie von speziellen Wundauflagen und Wundverbänden, versucht wird, eine Heilung der Wunde trotz fortbestehender Grunderkrankung zu erreichen.

Das ständig zunehmende Angebot an Therapeutika (Salben, Tinkturen, Puder, Antiseptika, Antibiotika) sowie Verbandmaterialien und Verbandstoffsystemen zur Behandlung chronischer Wunden, und das damit verbundene Problem der richtigen Auswahl und Anwendung, führt zu einem therapeutischen Vorgehen, das durch eine große Zahl verschiedener, unkoordinierter und oft wirkungsloser ärztlicher Maßnahmen gekennzeichnet ist (-Polypragmasie) und oft die Ursache für Heilungsverzögerungen und Kostensteigerungen ist.

### 3. Problem der Sterilisation, Dekontamination und Aufbereitung medizintechnischer Produkte:

Da für eine steigende Zahl medizintechnischer Produkte der Einsatz der bekannten Standard-Verfahren zur thermischen, chemischen, UV- und γ-Sterilisation aus materialtechnischen, konstruktionsspezifischen sowie umwelt- und gesundheitsrelevanten Gründen nicht mehr eingesetzt werden können, gewinnt die Entwicklung von Verfahren zur materialschonenden und effektiven antimikrobiellen Behandlung zunehmend an Bedeutung. Interessante Möglichkeiten zur Lösung dieses Problems bieten plasmatechnische Verfahren. Wie aus zahlreichen Publikationen und Patentschriften hervorgeht, wird weltweit intensiv an Lösungen dieser Art gearbeitet.

Durch den Einsatz von Plasmen zur materialschonenden Dekontamination von medizintechnischen Produkten aus empfindlichen, thermolabilen Materialien sind zwar bereits in einigen Fällen Entkeimungserfolge in der Größenordnung der für die Sterilisation geforderten 6 log-Stufen erzielt worden, allerdings nur für einige der relevanten Mikroorganismen. Die Möglichkeiten dieser Technik sind aber bisher noch nicht voll ausgeschöpft.

### [Aufgabe der Erfindung]

Der Erfindung lag die Aufgabe zugrunde, die Nachteile der im Stand der Technik beschriebenen Lösungen zu beseitigen.

### Lösung der Aufgabe

Die Aufgabe wurde gemäß den Merkmalen der Patentansprüche gelöst.

Der Erfindung liegt die Idee zugrunde, zur Erhöhung der Wirkung von Atmosphärendruckplasmen auf lebende Zellen die Zellen gleichzeitig zu elektroporieren. So werden bei der Anwendung für die Krebstherapie die Membranporen der Krebszellen eines erkrankten Hautareals durch impedanzgesteuerte Elektroporation selektiv geöffnet, so dass die elektroporierten Krebszellen durch die im Atmosphärendruckplasma (z.B. Plasmajet oder Oberflächen-Barrierenentladung) erzeugten Radikale abgetötet werden können und auf den zusätzlichen Einsatz von Chemotherapeutika verzichtet werden kann.

Auf der Grundlage dieser Erfindung ist es möglich, folgende Probleme zu lösen:
1. Problem der Krebstherapie: lokale, selektive Abtötung von Krebszellen ohne den Einsatz von Chemotherapeutika,
2. Problem der Heilung chronischer Wunden: neuer therapeutischer Ansatz zur Verbesserung der Wundbehandlung als Kombination einer antiseptischen Wirkung mit einer Stimulierung der Neubildung von gesundem Gewebe,
3. Problem der Sterilisation, Dekontamination und Aufbereitung medizintechnischer Produkte: verbesserte antimikrobielle Plasmawirkung durch Kombination mit einer Elektroporation der Mikroorganismen-Zellen.

Im Fall der Behandlung der Zellen von chronischen Wunden kann durch die parallele Anwendung von Elektroporation und Atmosphärendruckplasma bei optimaler Wahl der Behandlungsparameter eine wirksamere antiseptische Wirkung des Plasmas bei gleichzeitiger Stimulierung der Neubildung von gesunden Gewebezellen erzielt werden.

Das Problem der Sterilisation wird dadurch gelöst, dass durch eine reversible Elektroporation bei gleichzeitiger Plasmawirkung die Zellmembranporen der abzutötenden Mikroorganismen temporär geöffnet werden und dadurch eine effektivere Abtötung der Zellen durch die im Plasma erzeugten Radikale erfolgen kann.

Im Fall einer Anwendung zur Krebstherapie wird durch den Verzicht auf den Einsatz teurer Chemotherapeutika eine beträchtliche Senkung der Behandlungskosten erreicht und die Behandlung ist frei von Nebenwirkungen.

Bei der Behandlung von chronischen Wunden besteht der Vorteil der Erfindung in der Möglichkeit, von einer polypragmatischen Behandlung zu einer effizienten und kostengünstigeren, universell anwendbaren Behandlung zu gelangen, durch die eine effektive antiseptische Wirkung erzielt wird, die mit der Stimulierung einer Neubildung von gesundem Gewebe verbunden ist.

Durch die zusätzliche Anwendung einer Elektroporation während der Sterilisation, Dekontamination oder Aufbereitung medizintechnischer Produkte mittels Atmosphärendruckplasmen wird eine wesentliche Verbesserung der Sterilisationseffizienz erzielt.

### Beschreibung der Zeichnungen

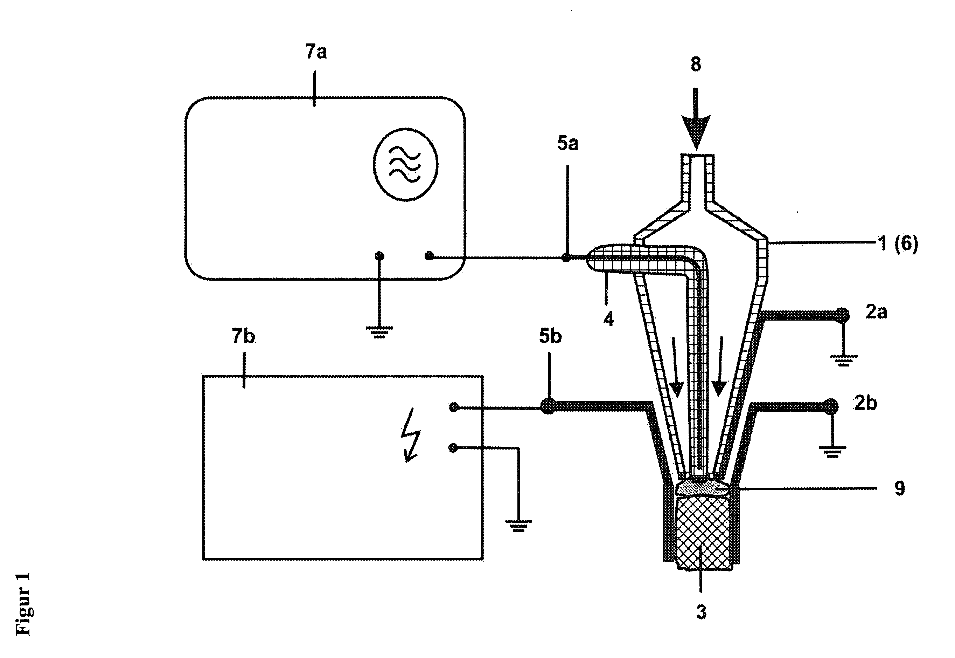

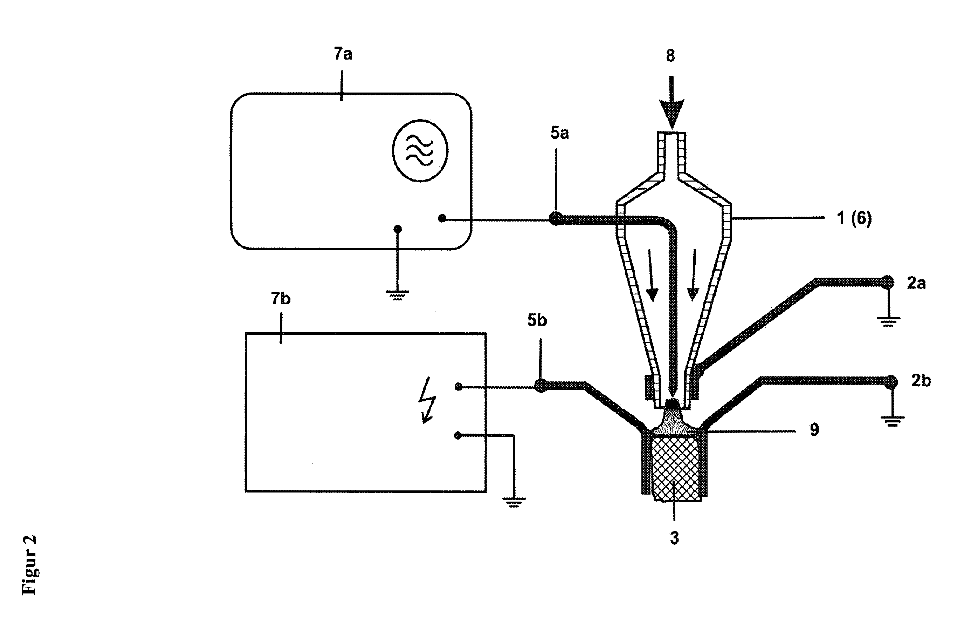

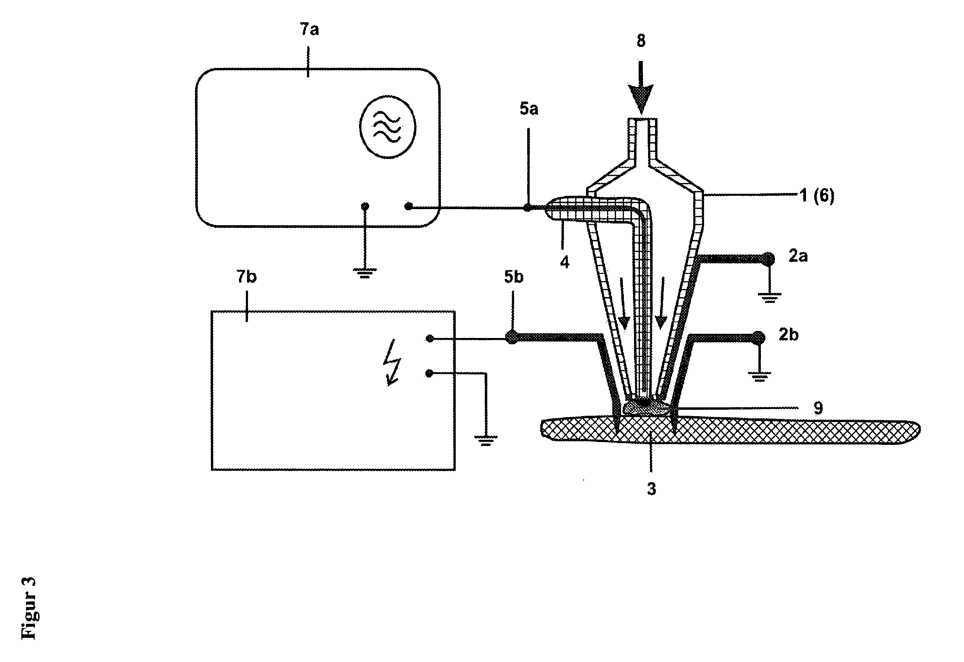

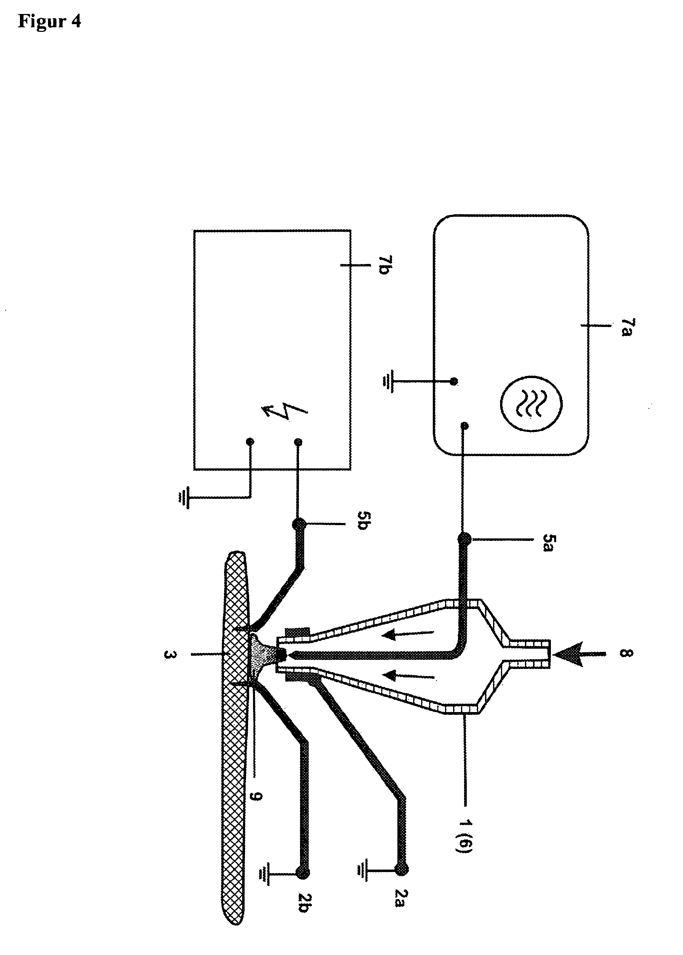

Die Erfindung wird anhand von Zeichnungen näher erläutert, ohne sie auf diese Zeichnungen zu beschränken. Die Fig. 1 bis Fig. 4 zeigen den prinzipiellen Aufbau der erfindungsgemäßen Vorrichtung beispielhaft anhand von vier Ausführungsformen, wobei bei den in Fig. 1 und Fig. 2 dargestellten Beispielen plattenförmige (2b und 5b) und im Fall von Fig. 3 und Fig. 4 nadelförmige Elektroporations-Elektroden verwendet werden. In den in Fig. 1 und Fig. 3 dargestellten Beispielen werden Oberflächen-Barrierenentladungen und in den in Fig. 2 und Fig. 4 gezeigten Anordnungen Plasmajets zur Plasmabehandlung verwendet.

### Bezugszeichenliste

Für die nachfolgenden Zeichnungen werden folgende Bezugszeichen verwendet:

| | | | |
|---|---|---|---|
| 1 | Gasdüse | 2 | geerdete Elektrode |
| 2a | geerdete Plasmaquellen-Elektrode | 2b | geerdete Elektroporations-Elektrode |
| 3 | Zellgewebe | 4 | Dielektrikum |
| 5 | Hochspannungs-Elektrode | 5a | Plasmaquellen-Elektrode |
| 5b | Elektroporations-Elektrode | 6 | Isolation |
| 7 | Spannungsversorgung | 7a | Plasmaquellen-Spannungsversorgung |
| 7b | Elektroporations-Spannungsversorgung | | |
| 8 | Gaszufuhr | 9 | Plasma |

## Patentansprüche

1. Vorrichtung zur Behandlung von lebenden Zellen mittels eines Plasmas bei gleichzeitiger Elektroporation der Zellen, **dadurch gekennzeichnet, dass** die Vorrichtung neben herkömmlichen Einrichtungen zur Elektroporation Einrichtungen zur Erzeugung eines Plasmas aufweist.

2. Vorrichtung nach Anspruch 1, **dadurch gekennzeichnet, dass** es sich um ein Atmosphärendruckplasma, vorzugsweise ein kaltes oder Nicht-thermisches Atmosphärendruckplasma, handelt.

3. Vorrichtung nach Anspruch 1 oder 2, **dadurch gekennzeichnet, dass** sie mindestens eine Plasmaquellen-Elektrode, mindestens eine Elektroporations-Elektrode, eine Gaszufuhr und mindestens eine Spannungsversorgung enthält.

4. Vorrichtung nach Anspruch 3, **dadurch gekennzeichnet, dass** sie zusätzlich eine Gasdüse, mindestens ein Dielektrikum, Isolation und mindestens eine Hochspannungs-Elektrode enthält.

## Claims

1. A device for treatment of live cells by means of a plasma at simultaneous electroporation of the cells, **characterised in that** the device apart from conventional means for electroporation comprises means for generation of a plasma.

2. The device according to claim 1, **characterised in that** the plasma is an atmospheric pressure plasma, preferably a cold or non-thermal atmospheric pressure plasma.

3. The device according to claim 1 or 2, **characterised in that** it comprises at least a plasma source electrode, at least an electroporation electrode, a gas supply and at least a voltage supply.

4. The device according to claim 3, **characterised in that** it comprises in addition a gas nozzle, at least a dielectric, insulation, and at least a high voltage electrode.

## Revendications

1. Dispositif pour le traitement des cellules vivantes au moyen d'un plasma avec une électroporation simultanée des cellules, **caractérisé en ce que** le dispositif outre des moyens conventionnels pour l'électroporation comprend des moyens pour la génération d'un plasma.

2. Dispositif selon la revendication 1, **caractérisé en ce qu'**il s'agit d'un plasma à pression atmosphérique, de préférence d'un plasma à pression atmosphérique froid ou non thermique.

3. Dispositif selon la revendication 1 ou 2, **caractérisé en ce qu'**il comprend au moins une électrode de source de plasma, au moins une électrode d'électroporation, un approvisionnement en gaz et au moins une alimentation en tension.

4. Dispositif selon la revendication 3, **caractérisé en ce qu'**il comprend en outre une buse à gaz, au moins un diélectrique, d'isolement et au moins une électrode à haute tension.
